Europäisches Patentamt

**European Patent Office**

Office européen des brevets

(19)

(11) Publication number: **0 205 293**

**A2**

(12) **EUROPEAN PATENT APPLICATION**

(21) Application number: **86304163.8**

(22) Date of filing: **02.06.86**

(51) Int. Cl.⁴: **A 61 K 39/395**
**A 61 K 49/00**

(30) Priority: **31.05.85 NZ 212274**

(43) Date of publication of application:
**17.12.86 Bulletin 86/51**

(84) Designated Contracting States:
**DE FR GB NL**

(71) Applicant: **Heslop, Barbara Farnsworth**
**1 Hart Street**
**Dunedin(NZ)**

(71) Applicant: **Bradley, Mark Philip**
**14 Mataora Road**
**Dunedin(NZ)**

(72) Inventor: **Heslop, Barbara Farnsworth**
**1 Hart Street**
**Dunedin(NZ)**

(72) Inventor: **Bradley, Mark Philip**
**14 Mataora Road**
**Dunedin(NZ)**

(74) Representative: **Silveston, Judith et al,**
**ABEL & IMRAY Northumberland House 303-306 High**
**Holborn**
**London, WC1V 7LH(GB)**

(54) **Improvements in or relating to methods of producing antibodies.**

(57) The invention relates to a method of stimulating the cells of the immune system of a host to produce antibodies and/or effector cells specific to the male antigen(s), to the antibodies and/or effector cells produced and to the use thereof in sex determination. In particular, the method includes positioning an implant carrying male antigen(s) within or in contact with the splenic substance of the host.

FIG 1.

EP 0 205 293 A2

This invention relates to a method of obtaining antibodies and/or effector cells of the immune system, to the antibodies and/or effector cells thus obtained, and to the use thereof. More particularly, it relates to methods of producing antibodies which react to the male antigen(s) and to the use of such antibodies in the sexing of embryos and sperm.

The type of antibody which is the subject of the invention is ordinarily identified as the H-Y antibody produced in response to the H-Y antigen, a cell surface component first identified as a histocompatability antigen responsible for the rejection of the male skin grafts by otherwise syngeneic female mice (1). Serological test developed in the 1970's which were presumed to identify the same antigen as that causing graft rejection indicated that the antigen was widely conserved, and that it characterised the heterogametic sex (2). However, more recent work has cast doubt upon the relationship between the H-Y antigen and the serologically determined male (SDM) antigen (3). Accordingly, the antibodies produced in accordance with the method of the invention are referred to herein as "male-specific antibodies" and include both those produced in response to the H-Y antigen and those produced in response to the SDM antigen.

Similarly, as used herein, the term "the male antigen(s)" is intended to include both the H-Y antigen and the SDM antigen.

The use of the male antigens and in particular the H-Y antigen in sex determination is an area where advancement has been dependent upon mastering the serology of the immunological response of a host to such antigens. The usual procedure has been to immunize female hosts (mice) by intraperitoneal or intrapedal injections of syngeneic male cells over an extended period of time. These conventional immunization protocols however have produced low titres of antibodies which have been difficult to identify (4,5). Due to these difficulties, the antibodies thus produced have been inferior diagnostic agents in sex determination.

It is therefore an object of the present invention to go some way towards overcoming the above disadvantages or at least to provide the public with a useful choice.

Accordingly, in a first aspect the invention may broadly be said to consist in a method of stimulating those cells of the immune system which react to the male antigen(s) in order to obtain antibodies and/or effector cells comprising

positioning an implant comprising male antigen(s) from a male donor presented on a physiologically-acceptable carrier within or in contact with the splenic substance of a female host such that the implant is accessible to the cells of the immune system of said host;

allowing the immune system of the host to respond immunologically to the male antigen(s); and

obtaining antibodies and/or effector cells of the immune system from the blood, spleen, lymph nodes or bone marrow of the said host,

the donor/host relationship being such that the donor and host are animals histoincompatible with respect to the male antigen(s).

In a second aspect the invention may be said to consist in a method of sexing mammalian embryos comprising the steps of

labelling the antibodies prepared in accordance with the method as defined above;

treating the embryo prior to implantation with said antibodies; and

detecting the presence or absence of said antibodies in said embryo.

In a third aspect, the invention may be said to consist in a method of sex determination comprising treating live semen with antibodies prepared in accordance with the method as defined above and separating the sperm thus treated into those which have taken up the antibodies and those which have not.

0205293

In a fourth aspect, the invention may be said to consist in a diagnostic kit for use in sex determination comprising antibodies produced according to the method as defined above and a label for said antibodies.

Various aspects of the of the present invention will be more clearly understood by reference to the accompanying drawings which are more fully described hereinafter and wherein

Figure 1 illustrates the results of an ELISA assay demonstrating the quantitative absorption of male-specific antibodies by sources of the male antigen(s)

Figures 2 and 3 show a comparison of the pattern of antibody response produced by the immunization protocol of the present invention as compared to conventional protocols in BN and HS female rats respectively.

Figure 4 shows the results of urease-ELISA with male-specific antibodies on caudal sperm flagellar plasma membranes.

Figure 5 shows the immunofluorescent localisation of male-specific antibodies over the plasma membranes of caudal spermatozoa.

In a first aspect, the present invention relates to a method of obtaining antibodies and/or effector cells by stimulating cells of the immune system of a female host which react to the presence of male antigen(s) from a male donor. The method is performed using a male donor and a female host which are histoincompatible with respect to the male antigen(s). In the preferred embodiment of the invention, the donor/host relationship is such that the donor and host are histoincompatible only with respect to the male antigen(s) but this is not critical. This relationship is conveniently achieved by the use of hosts and donors which are inbred laboratory rodents, for example inbred laboratory rats. Of course, other hosts such as rabbits, guinea pigs and dogs may also be used.

The first step of the method according to the present invention comprises positioning an implant comprising the male antigen(s) presented on a physiologically-acceptable carrier within or in contact with the splenic substance of the host such that the implant is accessible to the cells of the immune system of the host. The implant can be have a variety of forms. In one embodiment, the implant comprises cells obtained from a male donor. In this embodiment, the cells are preferably in a form such that they do not migrate from the spleen once they have been positioned within or in contact with the splenic substance. Conveniently, this is achieved by providing the donor cells in the form a single cell suspension, a suspension of dispersed cells or a cellular aggregate. In the presently preferred form, the donor cells are in the form of a skin graft which is positioned such that the cut surface of the graft is accessible to the cells of the immune system.

In a further embodiment, the implant may comprise a source of purified male antigen(s) coated onto the surface of a pharmaceutically-acceptable material. Conveniently, this material is a polystyrene bead, a latex particle or an albumin microsphere but of course other pharmaceutically acceptable carriers may be used.

The implant is positioned within or in contact with the splenic substance of the female host by breaching the splenic capsule. Subsequent to breaching the splenic capsule, the implant can be positioned in contact with the splenic substance either at the point at which the breach occurs or, as is presently preferred, deep within the substance of the spleen (intrasplenically). Conveniently, the positioning of the implant as above is achieved by the use of a hollow syringe needle in combination with a stylet but this is of course not critical. Further, although the implant can be positioned in contact with the splenic substance at one site only it can also be positioned at more than one site in order to increase the exposure of the male antigen(s) to the cells of the immune system and the consequent stimulation of effector cells and production of antibodies.

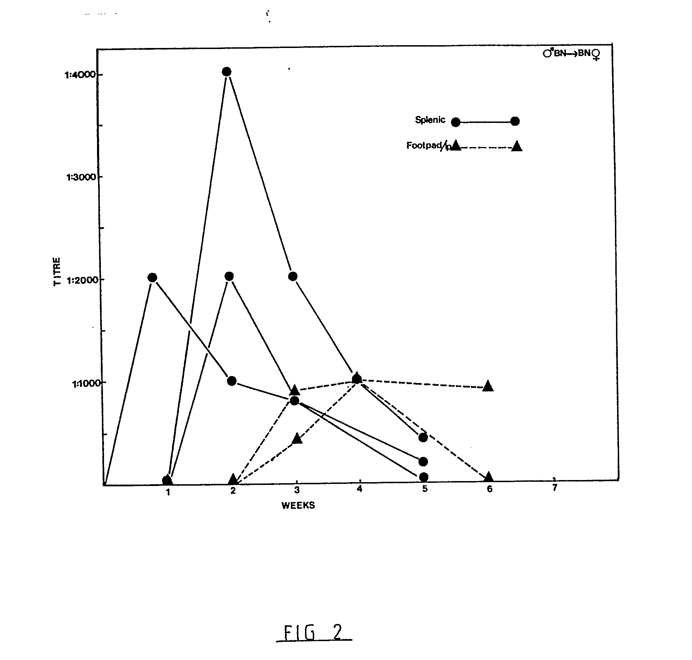

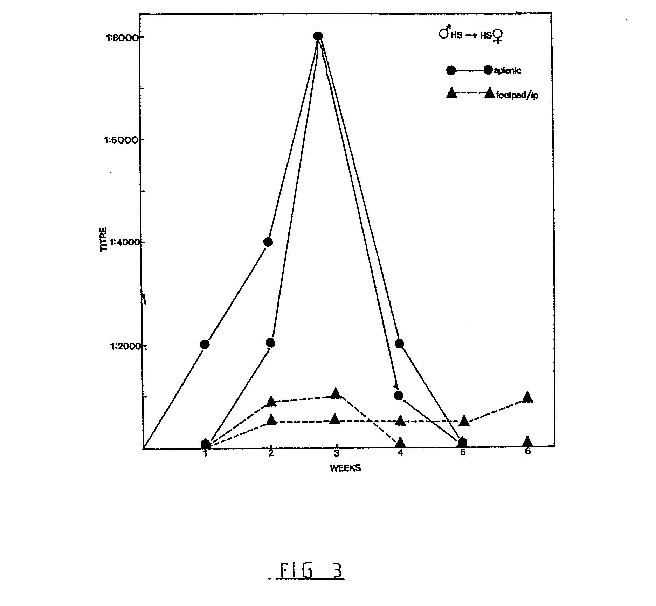

The second step of the method of the invention comprises allowing the immune system of the female host to respond immunologically to the male antigen(s) in order to stimulate effector cells leading to the production of antibodies. Experimental trials have found that the titre of effector cells produced will be greatest a certain period subsequent to the positioning of the implant within or in contact with the splenic substance of the host. This period will however vary depending on the type of implant, on the amount of male antigen and on the type of host. By way of example, in the specific example of the performance of the method of the invention described hereinafter, the antibody titre for certain strains of rats peaks from one to three weeks subsequent to the positioning of the implant (see Figs 2 and 3).

The third step of the method according to the present invention comprises obtaining antibodies and/or effector cells from the female host by extracting antibodies from the blood or body fluids and effector cells from the blood, spleen, lymph nodes or bone marrow. Conveniently, the antibodies are harvested from collected blood using conventional procedures.

The effector cells are also harvested using conventional procedures, for example by separation from anticoagulated blood over a density gradient.

The method of the invention described above may also include the preliminary step of preparing an implant in a form suitable for positioning within or in contact with the splenic substance of the female host. In the preferred form, where the implant is a skin graft, the skin graft is prepared having a high percentage of epidermis relative to the dermis. This is conveniently achieved by performing a pinch graft.

In one embodiment of the invention, the effector cells obtained are male-specific antibodies suitable for use in sex determination. In an alternative embodiment the effector cells of the immune system obtained are lymphocytes manufacturing male-specific antibodies. The lymphocytes making such antibodies are then transformed so that they become immortal in cell culture. A number of methods are known in the art for performing this transformation, including fusing the lymphocytes with myeloma cells (9), or exposing them to a suitable virus (as, for example, the exposure of human B lymphocytes to Epstein Barr Virus).

The polyclonal antibodies obtained according the method of the present invention are then preferably treated to remove auto-antibodies and other unwanted antibodies by selective absorption. Conveniently, this is achieved by selectively absorbing the antibodies with syngeneic female cells, preferably thymocytes or spleen cells.

Details of one specific embodiment of the method according to the present invention in order to produce male-specific polyclonal antibodies will now be described.

EXAMPLE 1: Production of male-specific antibodies

Two inbred rat strains were used in these experiments designated BN and HS respectively. The origin and characteristics of these animals are described in standard listings (6).

For the purposes of comparison for each strain two experimental groups of four female rats were used. The first group was immunized by the injection of $2 \times 10^7$ syngeneic male lymph node cells into the footpad. These cells were prepared as has been previously described (7) but without isotope labelling. The animals were then bled at weekly intervals and the serum assayed for the presence of male-specific antibodies. A booster injection of $2 \times 10^7$ lymph node cells was given to each rat intrapedally after each bleeding.

The second group of rats was immunized by implantation of syngeneic male skin into the spleen as follows. The rats were anaesthetized with ether, and the spleen was delivered through a subcostal incision. Pinch grafts of approximately one millimetre in diameter were prepared from a male skin donor by tenting the skin with a needle and slicing off the raised tissue. These grafts (4 per recipient) were loaded into a 18-gauge needle that was inserted into the spleen of each rat. The grafts were displaced in the appropriate position by use of a stylet as the needle was withdrawn. Bleeding from the puncture site was minimal and was controlled with a bacteriology swab. The abdominal incision was closed in two layers. The animals were each bled from the tail vein at weekly intervals and the serum assayed for the presence of male-specific antibodies.

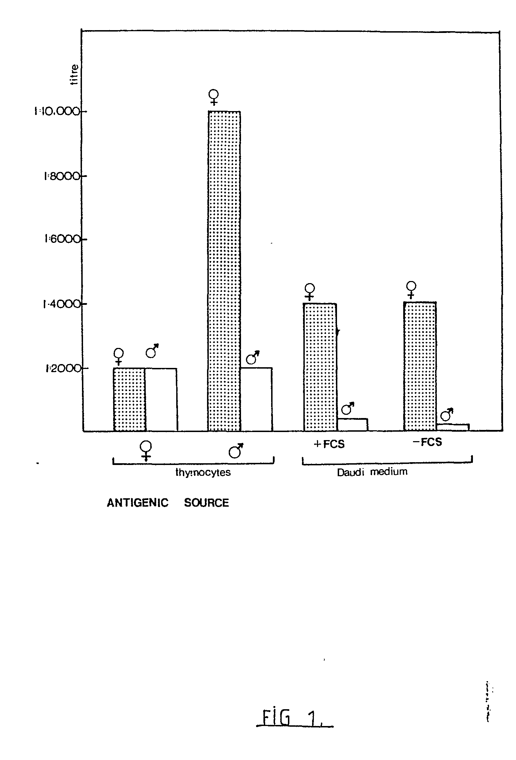

The assay of the serum obtained from each of the above groups was performed using a recently developed urease enzyme-linked immunosorbent assay (ELISA) protocol that used Daudi cell culture supernatant as the source of male antigen(s) (8). To demonstrate the specificity of the antiserum produced as above, the serum samples were first absorbed twice for two hours at·37°C with $10^8$ syngeneic female rate thymocytes to reduce the background noise from the possible presence of auto-antibodies in the serum. A sample of each serum was then subsequently absorbed in a similar manner with $10^8$ syngeneic male thymocytes to check the specificity of the response. The quantitative absorption data are presented in Figure 1.

Figure 1 shows that when female thymocytes were used as the antigenic source and probed with antiserum that had been preabsorbed with male or female thymocytes, high levels of non-specific reactivity (1:2000) were observed in the control assay. The specificity of the antiserum was demonstrated by the strong reaction by female-cell-absorbed antiserum to male thymocytes and the substantial decrease in antibody reactivity toward male thymocytes after this antiserum had been preabsorbed with male cells.

Daudi cell culture medium was found to be a better source of male-specific antigen for this particular assay because the level of background reactivity was substantially reduced (1:200) compared with that obtained when whole cells were used as the antigenic sources

- 9 -

0205293

(1:2000). Antibody titres were also reduced in this assay. Once again, the specificity of the antiserum was demonstrated by the reduction of antibody bonding after the serum had been preabsorbed with male cells.

The antibody production in intrasplenically-immunized BN female rates is shown in Figure 2 and compared with that resulting from conventional immunization protocols. The antibody production in accordance with the present invention is characterized by a rapid antibody response with titres reaching maximal values (1:2000-1:4000) in the first one to two weeks, and thereafter rapidly declining by the fifth week. In contrast, BN female rats conventionally immunized both intrapedally and intraperitoneally with male lymphocytes showed a slower antibody response with titres reaching a maximal level of between 1:500-1:1000 at three weeks and maintaining this level over the six week period of the experiment.

The pattern of H-Y antibody response in the intrasplenic immunized HS rats is shown in Figure 3. This response is similar to that observed for BN rats except that maximal titres were attained slightly later at three weeks and were higher (1:8000) than those for the BN strain. Once maximal antibody titres in the HS rats were reached in the third week the titres rapidly declined to zero by the sixth week. In the intrapedally immunized HS rats the antibody titres were lower than those recorded in the intrasplenic immunized rats (1:1000) and the time for the peak antibody response to occur was delayed. In this group of rats, these antibody titres were maintained over the ten-week experimental period, provided that the weekly booster injections were given.

It will be apparant from the above results that the intrasplenic immunization protocol according to the present invention is an effective method for generating high titre male-specific polyclonal antibodies. Furthermore, the advantages of the protocol according to the present invention can be readily appreciated by comparison with the titres of antibodies obtained using the conventional procedure of intrapedal or intraperitoneal injection of male cells.

It is also significant to note the transient nature of the antibody production using the method of the present invention and in particular the need to monitor carefully the level of antibody produced so that the serum can be harvested when the titre is at or towards the maximum. This unusual response is thought to arise because although the spleen is an unnatural site for an implant including male antigen(s), it is nevertheless regularly visited by numerous lymphocytes, and this in combination with the large number of likely antigen-presenting cells in skin provides very efficient exposure of antigen to the host immune system. Additionally the implant is weakly antigenic and so survives graft rejection for some time. All these factors combine to generate the environment which causes the very rapid build up in the titre of antibodies in the host animal.

The antisera thus obtained are appropriately treated to remove any auto-antibodies which may inadvertently be generated and are then able to be used in specified dilutions as a diagnostic tool for use in sex determination. In particular, the antibodies prepared according to the method described above can be used in the sexing of both mammalian embryos and sperm.

When used to sex mammalian embryos, or more correctly pro-embryos, the antibodies produced in accordance with the above method may be used either as whole antibody molecules or partial antibody molecules which contain the combining site. In the latter case, the method further includes the step of first cleaving the antibody molecule in order to obtain a partial antibody molecule, the $F(ab')_2$ fragment, containing the combining site. This is performed using conventional methods (10).

The sexing of mammalian embryos is performed by firstly labelling the the antibodies prepared as above. The antibodies can be labelled either directly or indirectly by known methods. By way of example, the method presently used by the applicants is to indirectly label the antibodies by treating them with a physiologically-acceptable fluorescent-labelled anti-immunoglobulin, or with $F(ab')_2$ fragments therefrom. This anti-immunoglobulin preferably has had the antispecies activity removed therefrom by absorbtion. The labelled anti-immunoglobulin presently used is the FITC anti-rat IgG fragment obtained from Cappel Laboratories in Pennsylvania, U.S.A., and designated 1713-3151. Of course, other methods of labelling the antibodies, either directly or indirectly such as using antibody-coated beads which can be detected visually, may be used.

Subsequent to treating the embryos with the labelled antibodies, the presence or absence of the antibodies in the embryo is detected. In the preferred embodiment, where the physiologically-acceptable fluorescent-labelled anti-immuoglobulin is used as the label, the attachment of the antibody to any particular embryo is detected visually by the use of a fluorescent microscope. However, as stated above, numerous methods of labelling antibodies are known in the art and accordingly the method used to detect the presence or absence of the labelled antibody will vary depending on the type of label used.

To illustrate the effectiveness of the antibodies of the invention in sexing embryos, a number of embryo sexing trials were performed on both rat and sheep embryos. The results of these trials are summarised in the following table.

| Rat 233 embryos in 10 trials | | | Sheep 47 embryos in 2 trials | | |
|---|---|---|---|---|---|
| male | female | uncertain | male | female | uncertain |
| 112 48% | 114 49% | 7 3% | 22 47% | 24 51% | 1 2% |

The technique demonstrated a clearly discernable difference between two groups embryos and consequently is considered to a powerful diagnostic method of sexing embryos.

In further experiments, a number of rat embryos were sexed in accordance with the above method prior to transplantation into female hosts for gestation. Up to the time of filing of the application, two of these hosts had given birth to live young. Both of the rats born were of the sex predicted prior to transplantation of the embryos.

The determination of the sex of sperm is performed by treating live semen with antibodies prepared in accordance with the present invention. The semen thus treated is separated into those sperm which have taken up the antibodies and those which have not by using any one of a number of conventional methods. By way of example, the antibody may be attached to microspheres prior to treatment of the semen, the semen being subsequently separated into those which have taken up the antibodies and those which have not by sedimentation. By way of further example, the semen thus treated may be separated by use of immunoaffinity methods, by chromotography, or by electrophoresis.

Experimental trials using the male-specific antibodies according to the invention to sex sperm were performed as follows:

Fresh testes from slaughtered mature rams were collected from a local abattoir. The epididymal tract was removed and spermatozoa from the caudal region were isolated by cutting this tissue into sperm isolating buffer (30mM Tris-HCl, ph 7.1, 103mM NaCl; 5mM KCl, 3mM MgCl$_2$,
0.4mM EGTA). The spermatozoa were washed twice in the same buffer by
centrifugation at 1000g for 10 min.

From the caudal ram spermatozoa obtained, flagellar plasma membranes
were prepared as previously described (11). These were stored at
-20°C until required.

Example 2: Male antigen detection in Flagellar Plasma Membranes by
ELISA

Purified flagellar plasma membranes were diluted 1:50 in ELISA
diluting buffer (PBS containing 0.24% BSA, 0.01% NaN$_3$). 30ul samples
were placed in each well of a NUNC immunoplate and incubated at 37 C
for 2hr. The plate was then washed with ELISA washing buffer (PBS pH
7.2, containing 0.05% Tween-20). Doubling dilutions of either female
or male absorbed male specific antibodies were than placed in the
appropriate wells and the plate was incubated for 1hr at 37 C. After
three washes with buffer 30ul of a 1:1000 dilution of urease-
conjugated anti-rat IgG was added to each well and the plate was incubated for a further 30min at 37 C. The plate was then washed three
more times with wash buffer followed by three washes with double-
distilled water before the addition of 30ul/well of urease substrate
(Commonwealth Serum Laboratories, Australia). Colour development was
recorded as a visual change from yellow to purple, and scored on an
arbitrary scale from 1 to 3.

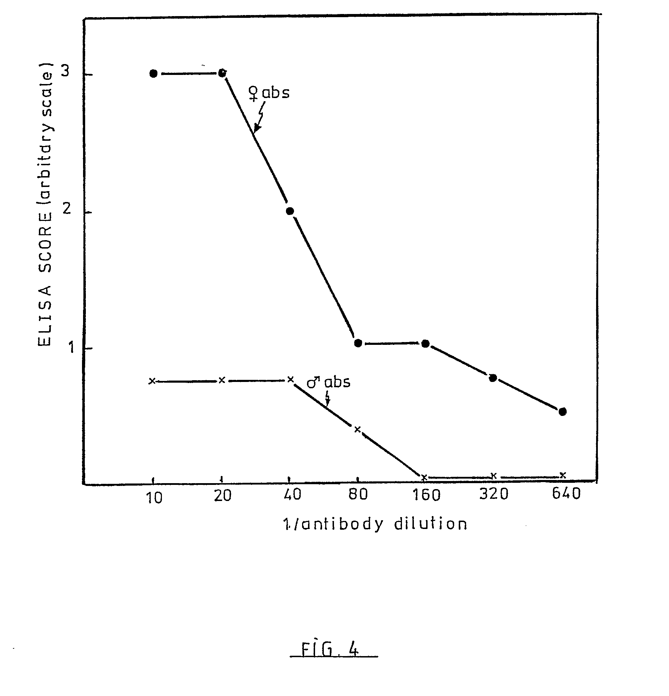

The results of this experiment are shown in Figure 4. Female absorbed
male-specific antiserum (•-----•) titred at 1:640. With the male
absorbed male-specific antiserum (o-----o) as a control for specificity
only a weak reaction was observed at a titre of 1:40. These results
clearly demonstrate that male antigen is present in flagellar plasma
membranes of caudal ram sperm to which the male-specific antibodies of
the invention attach.

Example 3:   Immunofluorescent labelling of Caudal Spermatozoa:

This experiment was designed to show the site of antiserum binding to the spermatozoa.

Washed caudal spermoatozoa were resuspended in a 1:200 dilution of male-specific antiserum in PBS and incubated for 2hr with gentle agitation at room temperature.  After a wash in PBS the cells were resuspended in a 1:10 dilution of FITC-anti-rat IgG [F(ab)$_2$] and incubated for 1hr.  Following two more washes with PBS the cells were examined by fluoresence microscopy and conventional light microscopy. Controls consisted of (i) spermatozoa treated as above except that the male-specific antiserum had been absorbed with male cells, and (ii) spermatozoa treated with FITC-labelled anti-rat IgG F(ab)$_2$ fragments but without prior exposure to the male-specific antiserum.

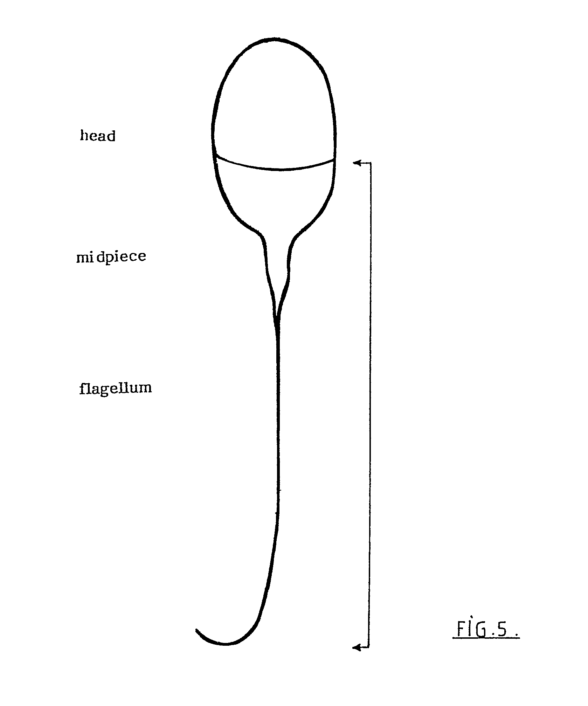

Figure 5 shows the site of attachment of the male-specific antibody of the invention.  The antibody attaches to the region between the arrows and can be detected by immunofluorescence.  With the caudal spermatozoa used in the experiment, fluorescence was detected in about 40% of the sperm.  No reaction occurs if the antiserum is previously absorbed with syngeneic male rat cells.  Thus, about 40% of ram sperm obtained from the caudal region of the epididymis appear to contain the male-specific antigen.

The antibodies produced in accordance with the present invention may also be used in a diagnostic kit for use in sex determination.  Such a kit comprises antibodies prepared according to the method of the invention together with a label for the antibodies.  Again, any one of a number of the labels known in the art may be used although a fluorescent-labelled anti-immunoglobulin is preferred.

It will be appreciated by those persons skilled in the art that above description of the invention is given by way of example only and that the scope of the invention is limited only by the appended claims.

- 15 -

# REFERENCES

(1) Eichwald EJ, Silmser CR. Untitled communication. _Transplant Bull_ 1955; 2:148.

(2) Silvers WK, Wachtel SS. H-Y antigen: behaviour and function. _Science_ 1977; 195:956.

(3) Fritz IB. The elusive H-Y antigen. _Cell_ 1983; 34:1.

(4) Koo SC. Serology of H-Y antigen. _Human Genetics_ 1981; 58:18.

(5) Ohno S. _Endocrine Reviews_ 1985, 6,421.

(6) Festing MFW. Inbred strains in biomedical research London: Macmillan, 1979.

(7) McNeilage LJ, Heslop BF. Lymphocyte homing in syngeneic and unsensitised MHC compatible allogeneic hosts. _Cell Immunol_ 1980; 50:58.

(8) Bradley MP, Heslop BF. An improved urease-ELISA protocol for the screening of monoclonal H-Y antibodies. _Proc Univ. Otago Med. Sch._ 1984; 62:14.

(9) Milstein C. _Monoclonal Antibodies in Clinical Medicine_ Ch 1 Eds. AJ McMichael and JW Fabre; Academic Press, London 1982.

(10) Stanworth DR and Turner MW. _Handbook of Experimental Immunology_ 3rd ed. Vol 1, Ch 6, Ed DM Weir, Blackwell Scientific Publications, Oxford 1978.

(11) Bradley MP and Forrester IT. A $[Ca^{2+} + Mg^{2+}]$-ATPase and active $Ca^{2+}$ transport in the plasma membranes isolated from ram sperm flagella. _Cell Calcium_ 1, 381-390 (1980).

CLAIMS:

1. A method of stimulating those cells of the immune system which react to the male antigen(s) in order to obtain antibodies and/or effector cells comprising

positioning an implant comprising male antigen(s) from a male donor presented on a physiologically-acceptable carrier within or in contact with the splenic substance of a female host such that the implant is accessible to the cells of the immune system of said host;

allowing the immune system of the host to respond immunologically to the male antigen(s); and

obtaining antibodies and/or effector cells of the immune system from the blood, spleen, lymph nodes or bone marrow of the said host,

the donor/host relationship being such that the donor and host are animals histoincompatible with respect to the male antigen(s).

2. A method according to claim 1 wherein the implant comprises cells obtained from a male donor.

3. A method according to claim 2 wherein the donor cells are retained within or in contact with the splenic substance by the use of donor cells in a form such that they do not migrate from the spleen once they have been positioned within or in contact with the splenic substance.

4. A method according to any one of claims 2 to 3 wherein the donor cells are in the form of a skin graft and the graft in positioned such that the cut surface of said graft is accessible to the cells of the immune system of the host.

5. A method according to any one of the preceding claims wherein the implant is positioned within in or in contact with the splenic substance of the host at more than one site.

6. A method according to any one of the preceding claims including the preliminary step of preparing the implant in a form suitable for positioning within or in contact with the splenic substance.

7. A method according to any one of the preceding claims wherein said implant is positioned intrasplenically.

8. A method according to any one of the preceding claims wherein antibodies suitable for use in sex determination are obtained.

9. A method according to any one of claims 1 to 7 wherein the effector cells of the immune system obtained are lymphocytes which are capable of being transformed to make monoclonal antibodies.

10. A method according to claim 9 further including the step of transforming the lymphocytes to make monoclonal antibodies suitable for use in sex determination.

11. A method according to any one of claims 8 and 10 including the further step of treating the antisera obtained by selective absorption to remove auto-antibodies and other unwanted antibodies.

12. A method sexing mammalian pro-embryos comprising the steps of
labelling the antibodies prepared in accordance with the method of any one of claims 8 and 10;
treating the embryo prior to implantation with said antibodies; and
detecting the presence or absence of said antibodies in said embryo.

13. A method according to claim 12 wherein the label is indirectly attached to said antibodies by treating said antibodies with a physiologically-acceptable fluorescent-labelled anti-immunoglobulin or a fragment thereof.

- 18 -

14. A method according to claim 13 wherein the anti-immunoglobulin is detected visually by the use of a fluorescent microscope.

15. A method of sex determination comprising treating live semen with antibodies prepared in accordance with the method of any one of claims 8 and 10 and separating the sperm thus treated into those which have taken up the antibodies and those which have not.

16. A diagnostic kit for use in sex determination comprising antibodies produced according to the method of any one of claims 8 and 10 and a label for said antibodies.

17. A diagnostic kit according to claim 16 wherein the label comprises a fluorescent labelled anti-immunoglobulin or a fragment thereof.

18. Antibodies suitable for use in sex determination produced in accordance with the method of any one of claims 8 and 10.

19. Lymphocytes for use in the production of monoclonal antibodies produced in accordance with the method of claim 9.

0205293

ANTIGENIC   SOURCE

FIG 1.

FIG 2

3/5

FIG 3

0205293

FIG. 4

0205293

head

midpiece

flagellum

FIG.5.